# EUROPEAN PATENT APPLICATION

(11) **EP 4 589 012 A1**
(43) Date of publication of application: **23.07.2025**
(21) Application number: 22958344.8
(22) Date of filing: 13.09.2022
(51) Int. Cl.: C12Q 1/68

(54) **SEQUENCING METHOD, PROCESSING SYSTEM AND SEQUENCING SYSTEM**

(71) Applicant: Qingdao MGI Tech Co.,Ltd, Shandong, 266000 (CN)
(72) Inventor: SHI, Xing, Shenzhen, Guangdong 518083 (CN); HUANG, Fuxing, Shenzhen, Guangdong 518083 (CN)
(74) Representative: Zacco GmbH
(86) International application number: PCT/CN2022/118410
(87) International publication number: WO 2024/055149

(57) **Abstract**

Disclosed is a sequencing method for a semiconductor sequencing chip, a system for processing data and a system for sequencing gene. The semiconductor sequencing chip includes a plurality of core row formed by arranging a plurality of cores. The sequencing method includes: the semiconductor sequencing chip is controlled to be immersed in a reagent used for each round of sequencing reaction in a mode taking the core row as an immersion unit, and a corresponding base is enabled to emit light or not to emit light when the semiconductor sequencing chip is immersed in the substrate reagent; each time when one unit of core row is immersed in the substrate reagent, data output by the core row immersed in the reagent is read at least once until all the core rows are immersed in the substrate reagent, a target template is determined according to the data output by the first core row, a signal range that whether the base emits light or not is defined, and data output by the remaining core rows is simplified by the target template to obtain optical signal data; and the type of the base is determined according to the optical signal data.

## Description

### Technical Field

The present disclosure relates to the technical field of gene sequencing, in particular to a sequencing method, a system for processing data and a system for sequencing gene.

### Background

Sequencing substrates (also called semiconductor sequencing chips) used by an existing high-throughput gene sequencer are generally of two types, including a surface chip and a semiconductor sequencing chip integrated circuit. The former generally captures a fluorescent signal by a microscope optical system, and the semiconductor sequencing chip completes acquisition and analog-to-digital conversion of an electrical signal or an optical signal through an internal integrated circuit.

When the semiconductor sequencing chip is used for sequencing, signals output of all pixels are read and processed to obtain a picture, and a scatter diagram is clustered by a complex image processing algorithm, so that four different bases ATCG are identified. However, the image processing algorithm may consume a large amount of computing resources, and thus computing load becomes progressively unacceptable when high-throughput, even ultra-high-throughput data is generated.

### Summary

The present disclosure aims at least to solve one of the technical problems in the related art. To this end, the present disclosure provides a sequencing method, a system for processing data and a system for sequencing gene. The sequencing method is simple, so data processing amount may be greatly reduced, and further, data amount transmitted and processed by a back-end server may be greatly reduced.

Some embodiments of the present disclosure provide a sequencing method, which is used for a system for sequencing gene. A semiconductor sequencing chip includes a plurality of cores, which are arranged to form a plurality of core rows. The sequencing method includes the following operations.

The semiconductor sequencing chip is controlled to contact a reagent used for each round of sequencing reaction in a mode taking the core row as a contact unit, and a corresponding base is enabled to emit light or not to emit light when the semiconductor sequencing chip contacts the reagent.

Each time when N units of core rows contact the reagent, N>0, data output by the core row contacting the reagent is read at least once until all the core rows contact the reagent.

After data output by the first core row is read, a target template is determined according to the data output by the first core row, and the target template includes a signal range that whether the base emits light or not.

Data output by the remaining core rows is simplified according to the target template to obtain optical signal data of different bases.

The base type is determined according to the optical signal data.

In the above sequencing method, the core row is contacted in a contact mode taking the core row as a contact unit, the target template is determined according to the data output by the first core row, then the data output by the remaining core rows is simplified to obtain optical signal data of different bases, data processing amount of the remaining core rows may be greatly reduced, and further data amount transmitted and processed may be greatly reduced.

Some embodiments of the present disclosure provide a system for processing data, which is used for a system for sequencing gene. The system for processing data includes a semiconductor sequencing chip, a control apparatus and a manipulator, the semiconductor sequencing chip includes a processing module and a plurality of cores, the plurality of cores are distributed in an array to form a plurality of core rows, the processing module is connected with the core row and the control apparatus, and the control apparatus is connected with the manipulator.

The control apparatus is configured to:
control the manipulator to enable the semiconductor sequencing chip to contact a reagent used for sequencing reaction in a mode taking the core row as a contact unit, and enable a corresponding base to emit light or not to emit light when the semiconductor sequencing chip contacts the reagent.

The processing model is configured to:
each time when N units of core rows contact the reagent, N>0, read data output by the core row contacting the reagent at least once until all the core rows contact the reagent.

A system for sequencing gene provided by some embodiments of the present disclosure includes the above system for processing data.

In the above system for processing data and system for sequencing gene, the core row is contacted in a contact mode taking the core row as a contact unit, the target template is determined according to the data output by the first core row, then the data output by the remaining core rows is simplified to obtain optical signal data of different bases, data processing amount of the remaining core rows may be greatly reduced, and further, data amount transmitted and processed may be greatly reduced.

Additional aspects and advantages of the present disclosure will be set forth in part in the description which follows, which will be obvious from the description which follows, or may be learned by practice of the present disclosure.

### Brief Description of the Drawings

The above and/or additional aspects and advantages of the present disclosure will become apparent and readily appreciated from the following description of the implementation modes taken in conjunction with the accompanying drawings.
Fig. 1 is a flowchart of a sequencing method according to some embodiments of the present disclosure.
Fig. 2 is a schematic block diagram of a system for processing data of a semiconductor sequencing chip according to some embodiments of the present disclosure.
Fig. 3 is a schematic block diagram of an image control module of a system for processing data of a semiconductor sequencing chip according to some embodiments of the present disclosure.
Fig. 4 is schematic diagram of a target template of a sequencing method according to some embodiments of the present disclosure.
Fig. 5 is a diagram showing correspondence between optical signal data and base type according to Some embodiments of the present disclosure.
Fig. 6 is a schematic structure diagram of a semiconductor sequencing chip according to some embodiments of the present disclosure.
Fig. 7 is a schematic diagram of region division of a semiconductor sequencing chip according to some embodiments of the present disclosure.
Fig. 8 is a structure diagram of a core of a semiconductor sequencing chip according to some embodiments of the present disclosure.
Fig. 9 is another schematic structure diagram of a core of a semiconductor sequencing chip according to some embodiments of the present disclosure.
Fig. 10 is still another schematic structure diagram of a core of a semiconductor sequencing chip according to some embodiments of the present disclosure.
Fig. 11 is a timing diagram of data reading according to some embodiments of the present disclosure.

Description of Reference Numerals:
Manipulator 10, Target template 11, Reagent tank 12, First signal range 13, Semiconductor sequencing chip 14,
Second signal range 15, Mechanical control module 16, Third signal range 17, Fluid control module 18,
Fourth signal range 19, Temperature control module 20, Environment control module 22, Image processing module 24,
Control apparatus 26, Display screen 28, Control module 30, Power board 32, Row switching and common reading unit 34,
Main control board 36, Driving board 40, Core 42, Core row 44, Region 48, Sub-pixel array 50,
Pixel array 51, Phase lock loop, Decoder controller and Digital buffer 52,
Correlated double sampling circuit and Comparator 54, Readout circuit 56, Decoder and Driver program 58, and
System for processing data 100.

### Detailed Description of the Embodiments

Embodiments of the present disclosure will be described in detail below, examples of the embodiments are illustrated in the drawings, in which same or similar reference numerals refer to the same or similar elements or elements having the same or similar function throughout. The embodiments described below by reference to the drawings are exemplary only for explaining the present disclosure and are not to be understood as limiting the present disclosure.

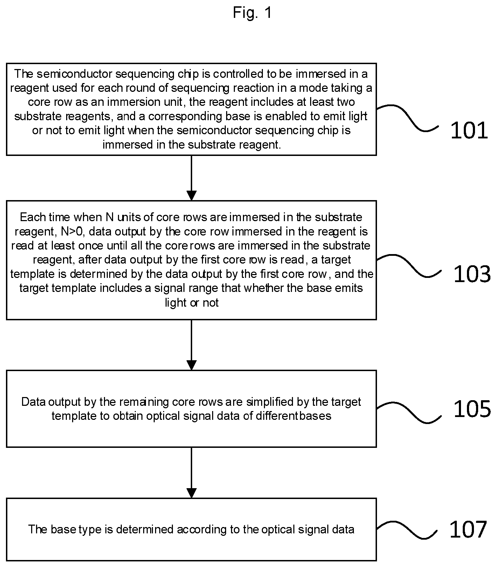

Referring to Figs. 1, 2 and 6, a sequencing method for a semiconductor sequencing chip 14 provided by some embodiments of the present disclosure is used for a system for sequencing gene. The semiconductor sequencing chip 14 includes a plurality of cores 42, and the plurality of cores 42 are arranged to form a plurality of core rows 44.

The semiconductor sequencing chip 144 may include a sequencing side and a back plate, or may include two sequencing sides, the sequencing side includes a core 42, and the back plate is the side which does not include the core 42. The sequencing side and a reagent may complete the purpose of contact by the action of "contact", and the action of contact may be performed in several ways, but is not limited to: the reagent realizes the purpose of contact with a fixed sequencing side in the modes including flowing, developing and the like; the purpose of contact is realized by immersing the sequencing side in the reagent in the modes including moving, rotating and the like; and the sequencing side and the reagent realize the purpose of contact in the mode of relative movement. All the above modes may realize contact of the sequencing side and the reagent, which should be understood as that replacing is carried out according to an actual use scene. For ease of explaining the essence of the present disclosure, the description will be given by taking "the purpose of contact is realized by immersing the sequencing side in the reagent in the mode of moving" as an example.

The sequencing method includes the following steps.

In S101, the semiconductor sequencing chip 14 is controlled to be immersed in a reagent used for each round of sequencing reaction in an immersion mode taking a core row 44 as an immersion unit, the reagent includes at least two substrate reagents, and a corresponding base is enabled to emit light or not to emit light when the semiconductor sequencing chip 14 is immersed in the substrate reagent.

In S103, each time when N units of core rows 44 are immersed in the substrate reagent (N>0), data output by the core row 44 immersed in the reagent is read at least once until all the core rows 44 are immersed in the substrate reagent.

After data output by the first core row 44 is read, a target template 11 is determined by the data output by the first core row 44, and the target template 11 includes a signal range that whether the base emits light.

In S105, data output by the remaining core rows 44 are simplified by the target template 11 to obtain optical signal data of different bases.

In S107, the base type is determined according to the optical signal data.

In the above sequencing method, the semiconductor sequencing chip 14 is immersed in a mode taking the core row 44 as an immersion unit, the target template 11 is determined by the data output by the first core row 44, and then the data output by the remaining core rows 44 is simplified to obtain optical signal data of different bases, so that data amount transmitted and processed may be greatly reduced.

The semiconductor sequencing chip 14 includes a plurality of cores 42, the plurality of cores 42 are arranged to form a plurality of core rows 44, and at the beginning of sequencing, base sequence clusters/spheres of different amplification starting fragments subjected to the same amplification process are loaded on the core row 44 immersed in the reagent at the earliest. In an example, there is only one core 42 in the first core row 44, and base sequence clusters/spheres of different amplification starting fragments (such as insert size 50 bp, 100 bp, 200 bp, and 300 bp) subjected to the same amplification process are loaded on the core 42. Through the step, one target template 11 may be obtained after biochemical reaction of the first core row 44 of the semiconductor sequencing chip 14.

In the sequencing process, the semiconductor sequencing chip 14 is immersed in a reagent used for each round of reaction in a mode taking the core row 44 as an immersion unit, the reagent includes at least two substrate reagents, when the semiconductor sequencing chip 14 is immersed in the substrate reagent, the substrate reagent enables different base types to show different light-emitting intensities, each time when N (N>0) units of core rows 44 are immersed in the substrate reagent, optical signal data output by the core row 44 is read, the data output by the remaining core rows 44 is simplified by the target template 11 to obtain optical signal data of different bases, and the base type may be determined through the optical signal data output by the semiconductor sequencing chip 14.

The immersion unit immersed in the reagent each time is N, and N represents the number of the core row 44 entering the reagent each time. When N=1, each time when one core row 44 is immersed, data output by the semiconductor sequencing chip 14 is read, N may be a numerical value greater than 1, in such a case, when N core rows 44 are immersed each time, data output by the semiconductor sequencing chip 14 is read once, so that the speed of data reading may be improved. Or for a device compatible with a slow reading speed, N=0.5, in such a case, when one core row 44 is immersed, data reading is carried out twice. The number of the core row 44 immersed in the reagent each time and the times of signal reading each time need to be set according to actual conditions, which are not particularly limited herein.

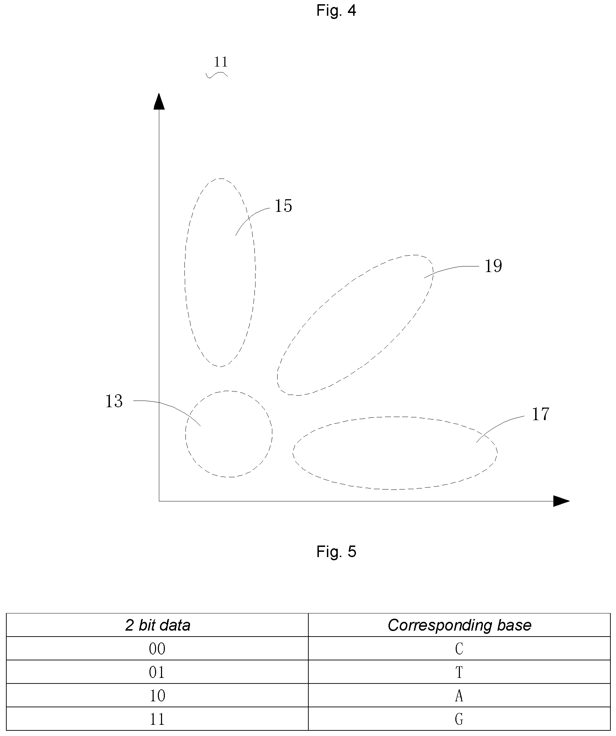

In some embodiments, the reagent includes two substrate reagents, after the first core row 44 of the semiconductor sequencing chip 14 outputs data of the two substrate reagents (namely, two channel data), one target template 11 may be obtained, as shown in Fig. 4, by utilizing that the target template 11 includes a signal range that whether the base emits light or not (the range defined by the dashed box in Fig. 4 ), the data output by the remaining core rows 44 is simplified to obtain optical signal data of different bases. The type of the base may be determined according to the output optical signal data.

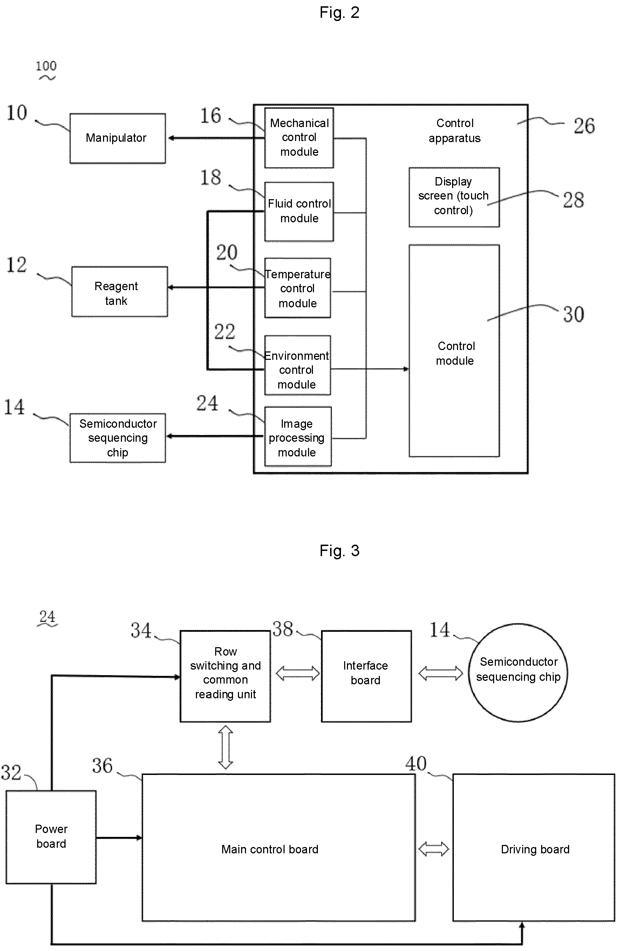

Referring to Fig. 2, a system for processing data 100 of the implementation mode of the present disclosure includes a semiconductor sequencing chip 14, a control apparatus 26 and a manipulator 10. The reagent may be placed in a reagent tank 12. The manipulator 10, the reagent tank 12 and the semiconductor sequencing chip 14 may be placed in closed space in which a series of biochemical reactions for gene sequencing are performed. One or more reagent tanks 12 are available, and each reagent tank 12 contains a reagent required for gene sequencing. Biochemical reactions occurring in the reagent tank 12 is required to be maintained at a certain temperature, time and environment, and these conditions are controlled by the control apparatus 26.

The semiconductor sequencing chip 14 includes a processing module (not shown) and a plurality of cores 42, the plurality of cores 42 are distributed in an array to form a plurality of core rows 44, the processing module is connected with the core row 44 and the control apparatus 26, after each unit of core row 44 is immersed in the substrate reagent, the processing module reads data output by the core row 44, a target template 11 is determined by data output by the first core row 44, the signal range whether the base emits light or not is limited by the target template 11, and data output by the remaining core rows 44 is simplified by the target template 11 to obtain and output optical signal data of different bases.

The control apparatus 26 may include a display screen 28 and a control module 30. The display screen 28 may be a touch display screen, and control over the whole gene sequencing process by an operator is realized through the display screen 28. The control module 30 may include a mechanical control module 16, a fluid control module 18, a temperature control module 20, an environment control module 22 and an image processing module 24.

The mechanical control module 16 is configured to control the manipulator 10 to clamp the semiconductor sequencing chip 14 to move, and control the semiconductor sequencing chip 14 to be immersed in a reagent in a reagent tank 12 in an immersion mode taking the core row 44 as an immersion unit, the mechanical control module 16 may control the reaction time of the semiconductor sequencing chip 14 in the reagent tank 12, meanwhile, the mechanical control module 16 may control the movement speed of the manipulator 10 within an appropriate value range, so that the amount of reagent taken out when the semiconductor sequencing chip 14 enters and leaves the reagent tank may be reduced, and in addition, the appropriate movement speed of the manipulator 10 may also reduce amount of bubbles produced in the reagent.

The fluid control module 18 is configured to monitor the content of the reagent in the reagent tank 12, and by monitoring quality of the reagent, the fluid control module 18 may monitor change of content of the reagent in the reagent tank, and then is responsible for controlling relevant water pump and valve to supplement and circulate the reagent, so as to maintain the content of a key reactant needed for biochemical reaction in the reagent at a certain level.

The temperature control module 20 is configured to monitor and control the temperature in the closed space to be maintained at the appropriate temperature required by biochemical reaction through a temperature sensor.

The environmental control module 22 is configured to monitor and control the content of various major gases in the closed space, by filling nitrogen or other means, to ensure that the biochemical reaction is carried out in a low oxygen environment.

Referring to Fig. 3, the image processing module 24 may be connected with the semiconductor sequencing chip 14 through an interface board 38, the image processing module 24 includes a row switching and common reading unit 34, a main control board 36 and a driving board 40, image signals collected by the semiconductor sequencing chip 14 are read out row by row by the row switching and common reading unit 34 and stored in a back-end hard disk, and the row switching and common reading unit 34 may also communicate with the main control board 36 for bidirectional data transmission. The power board 32 supplies power for the image processing module 24, and the main control board 36 carries out data collection, calculation and instruction output. The driving board 40 is connected with a display for output.

In some embodiments, the reagent includes a first substrate reagent and a second substrate reagent, when the semiconductor sequencing chip 14 is immersed in the first substrate reagent or the second substrate, two bases are enabled to emit light, and the other two bases do not emit light. Therefore, four different bases may be distinguished through light-emitting condition of two channels.

Alternatively, when the semiconductor sequencing chip 14 is immersed in the first substrate reagent or the second substrate reagent, biochemical reaction occurs in the first substrate reagent or the second substrate reagent, which shows as that the light-emitting intensities of the bases are different in the reaction, the signal range of light emission is limited by the target template 11, and then the light-emitting condition of the bases in the first substrate reagent or the second substrate reagent may be obtained for distinguishing different bases.

In some embodiments, referring to Fig. 4, the target template 11 includes a first signal range 13, a second signal range 15, a third signal range 17 and a fourth signal range 19, and the types of the base include a first type, a second type, a third type and a fourth type.

The first signal range 13 indicates that the base of the first type does not emit light in both the first substrate reagent and the second substrate reagent.

The second signal range 15 indicates that the base of the second type does not emit light in the first substrate reagent and emits light in the second substrate reagent.

The third signal range 17 indicates that the base of the third type emits light in the first substrate reagent and does not emit light in the second substrate reagent.

The fourth signal range 19 indicates that the base of the fourth type emits light in both the first substrate reagent and the second substrate reagent. Therefore, the specific base type may be determined in the biochemical reaction.

Alternatively, the target template 11 determined by the data output by the first core row 44 includes a first signal range 13, a second signal range 15, a third signal range 17 and a fourth signal range 19, four bases react in the first substrate reagent or the second substrate reagent to show different light-emitting intensities, two signals, namely, light emitting or no light emitting, are output through the signal range defined by the target template 11, meanwhile, the reaction result of each base in two substrate reagents is unique, and therefore, the type of the current base may be accurately determined.

In some embodiments, the sequencing method includes the following operation.

The data output by the remaining core rows 44 is classified into the signal range of the target template 11 by an intercept classification algorithm. Therefore, a sequencing error may be reduced, and a more accurate sequencing result is obtained.

Alternatively, a signal scatter diagram may be obtained on a coordinate axis through data read after the first core row 44 finishes reaction in the reagent, two coordinate axes represent light-emitting intensities of the base in two substrate reagents, scatter points in the coordinate axis are mostly concentrated in four regions according to different light-emitting conditions of the base, by analysis processing on four regions with concentrated scatter points, tracing is carried out along the scatter points of the outer ring of each concentrated region, and round (or oval) regions representing four different bases may be distinguished on the diagram, namely, the signal range defined by the target template 11, such as the first signal range 13, the second signal range 15, the third signal range 17 and the fourth signal range 19 shown in Fig. 4. When data of the remaining rows are read, the target template 11 obtained by the first core row 44 is used as a reference, the data output by the remaining core rows 44 is classified into the signal range of the target template 11 by the intercept classification algorithm, namely, by comparing the distance (namely, intercept) between the position of the read data on the coordinate and the center of each circle (or ellipse), the point is classified into the range of the circle (or ellipse) with the shortest intercept, then a sequencing error may be reduced, and a more reliable sequencing result may be obtained.

In some embodiments, simplifying processing includes binarization processing. Therefore, data calculation amount may be reduced, and meanwhile, a sequencing result is accurately output.

Alternatively, through signal range defined by the target template 11, read different light-emitting intensities of the base are simplified to output two signals, namely, light emitting or no light emitting, with "0" representing no light emitting, and "1" representing light emitting. Therefore, when the base reacts in two substrate reagents, optical signal data including light emitting or no light emitting may be output twice, marked as "00", "01", "10", or "11", and different optical signal data correspond to bases of different types, as shown in Fig. 5. In such a case, each pixel finally outputs one 1 bit data, a 2 bit data is obtained after two substrates react, the 2 bit data corresponds to one of four bases "AGCT", and thus data transmission and processing pressure may be greatly alleviated. It may be understood that in other implementation modes, simplifying processing may also be other simplifying processing, but is not limited to binarization processing, and simplifying processing may be understood as processing original data to reduce output of data amount. Binarization is also not limited to 0 and 1, and also may be represented by other numerical values or symbols.

In an example, the semiconductor sequencing chip 14 is loaded with base sequence clusters/spheres of the starting fragment subjected to the same amplification process row by row under control of the manipulator 10, and then is immersed in sequencing reagent in sequence with the core row 44 as an immersion unit, when the semiconductor sequencing chip 14 is immersed in the first substrate reagent with the core row 44 as the immersion unit, 1 bit optical signal data of all the core rows are read out, when the first core row of the semiconductor sequencing chip 14 is immersed in the second substrate reagent, all 2 bit data of the first core row of the semiconductor sequencing chip 14 is read, and thus a target template 11 is obtained.

When the second core row of the semiconductor sequencing chip 14 is immersed in the second substrate reagent, at the time, data of the second core row is classified into the signal range of the target template 11, 1 bit optical signal data output is combined with 1 bit optical signal data output when the second core row of the semiconductor sequencing chip 14 is immersed in the first substrate reagent to form 2 bit optical signal data "00", "01", "10", or "11" corresponding to one of four bases "AGCT", and then optical signal data of the base on the second core row of the semiconductor sequencing chip 14 may be obtained.

Along with that the semiconductor sequencing chip 14 is immersed in the second substrate reagent with the core row 44 as the immersion unit for biochemical reaction and data reading till all core rows 44 of the whole semiconductor sequencing chip 14 are immersed in the second substrate reagent and reading is completed, optical signal data of first bases of all base sequence clusters/spheres loaded on the semiconductor sequencing chip 14 are read out, and in such a case, one sequencing cycle is completed. Through the above sequencing process, a plurality of times of sequencing cycles are carried out until the optical signal data of each base on all base sequence clusters/spheres is read out, and one sequencing is completed.

In some embodiments, the system for sequencing gene includes a row switching and common reading unit 34, the row switching and common reading unit 34 is connected with all the core rows 44, and the sequencing method includes the following operation.

The row switching and common reading unit 34 controls data channel switching and data reading of the core 42 immersed in the reagent. In such a case, data of the semiconductor sequencing chip 14 may be read out row by row, and then the difficulty of reading, routing, transmission, buffering and data processing of a computer system is reduced.

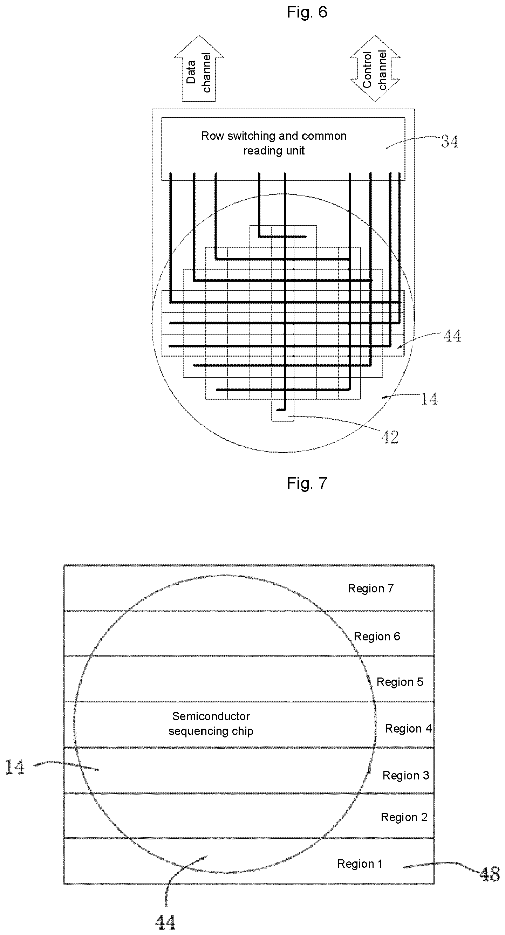

Alternatively, first, logical relationship among a plurality of chip cores 42 manufactured by an optical mask on a wafer is defined, in an example, referring to Fig. 6, 69 cores 42 are distributed on a semiconductor sequencing chip 14 in an array, by processing a circuit in the cores 42, 69 cores 42 are divided into 9 core rows 44, and one or more cores 42 on each unit of core row 44 read data at the same time. The row switching and common reading unit 34 may include a row switching and common reading circuit, the row switching and common reading unit 34 is connected with all the core rows 44, image signals are read row by row through the row switching and common reading unit 34, and under interaction of the control channel, data is read at least once each time at least one unit of core row 44 is immersed.

Namely, through joint control of immersion-reading, system load of sequential reading is reduced to 16% of that of parallel full wafer (semiconductor sequencing chip 14) reading. By adopting the above system logic and algorithm, four bases ATCG may be represented by compressing 2 bits from 10 bits of digital quantity of each pixel, through data processing on the core immersed first, after data of all the cores of the whole wafer (semiconductor sequencing chip 14) is normalized, a base sequence result may be obtained by a simplified algorithm. Therefore, the implementation of transmission, calculation and storage of the system is very simple and the cost is very low.

In some embodiments, the sequencing method includes the following operation.

**The** time, immersed in the reagent, of each unit of core row 44 is controlled to be the same. In such a case, it may ensure that a time difference of time of biochemical reaction of the core row 44 immersed each time and the reagent does not exceed a preset range.

Alternatively, in a signal generation system of biological self-luminescence or other enzymatic luminescence, the core 42 on the semiconductor sequencing chip 14 starts to generate a signal after contacting a substrate through enzyme carried by precursor biochemical reaction, with its signal curve strongly related to factors including temperature, time and the like. By setting movement time of the manipulator 10 in the mechanical control module 16, the manipulator 10 clamps the semiconductor sequencing chip 14 to be immersed in the reagent taking the core row 44 as an immersion unit, a time difference of time that each core 44 is immersed in the reagent is controlled to not exceed the preset range, so that the time of biochemical reaction occurring on each unit of core row 44 is uniform.

In some embodiments, the sequencing method includes the following operation.

The time difference of data reading time of each unit of core row 44 is controlled to not exceed a preset range. In such a case, it may be ensured that signals obtained by all the core rows 44 are relatively uniform.

Alternatively, the image processing module 24 is controlled to set same data reading time, the time difference of time of biochemical reaction occurring in the reagent of one unit of core row 44 immersed each time does not exceed a preset range, and a time collection signal fixed after each unit of core row 44 may ensure that signals obtained by all the core rows 44 are relatively uniform.

In some other implementation modes, it is possible that the data reading time set by the image processing module 24 does not exceed a preset time range, the time of biochemical reaction occurring in the reagent of the core row 44 immersed each time and the preset range determine the preset time range, the image processing module 24 collects a signal for the immersed part in the preset time range, and it may ensure that all the core rows 44 may collect data. For example, the preset range is 0-1 s, if the immersion time of the core row is 10 s, the preset time range of data reading time is 10 s-11 s, and then it may satisfy that the time difference of the immersion time of the core row and the data reading time does not exceed a preset range.

In some embodiments, the sequencing method further includes the following operation before the semiconductor sequencing chip 14 is immersed in the reagent.

The whole semiconductor sequencing chip 14 is divided into a plurality of regions 48 in a manner of parallel to a reagent tank 12, each region 48 contains one core row 44, and the correspondence between each region 48 and each unit of core row 44 is stored. In such a case, the amount and times of movement when the manipulator 10 clamps the semiconductor sequencing chip 14 to move each time are determined.

Alternatively, referring to Fig. 7, the whole semiconductor sequencing chip 14 is divided into a plurality of regions 48 in a manner of parallel to the reagent tank 12, each region 48 contains one core row 44, and each region 48 is the part where the manipulator 10 moves once to be immersed into the reagent to have biochemical reaction and read data. By dividing the plurality of regions 48, in the process of operating the whole semiconductor sequencing chip 14, the amount and times of movement when the manipulator 10 clamps the semiconductor sequencing chip 14 to move each time are determined. In the implementation mode shown in Fig. 7, the whole semiconductor sequencing chip 14 is divided into 7 regions.

In other implementation modes, the whole semiconductor sequencing chip 14 is divided into a plurality of regions 48, each region 48 may be a plurality of core rows 44, and by changing its circuit logic, the plurality of core rows 44 serve as one region 48 to have biochemical reaction and read data in an action of being immersed into the reagent controlled by the manipulator 10.

In some embodiments, the semiconductor sequencing chip 14 is controlled to be immersed into the reagent by the manipulator 10, continuous exposure time of each unit of core row 44 is controlled by a first time sequence, movement time of the manipulator 10 is controlled by a second time sequence, and a waiting time is separated before and after the continuous exposure time of each unit of core row 44 in the first time sequence from each movement time of the manipulator 10 in the second time sequence. In such a case, it is ensured that the manipulator 10 is in a static state during the exposure time, and an image signal is clearer and more accurate.

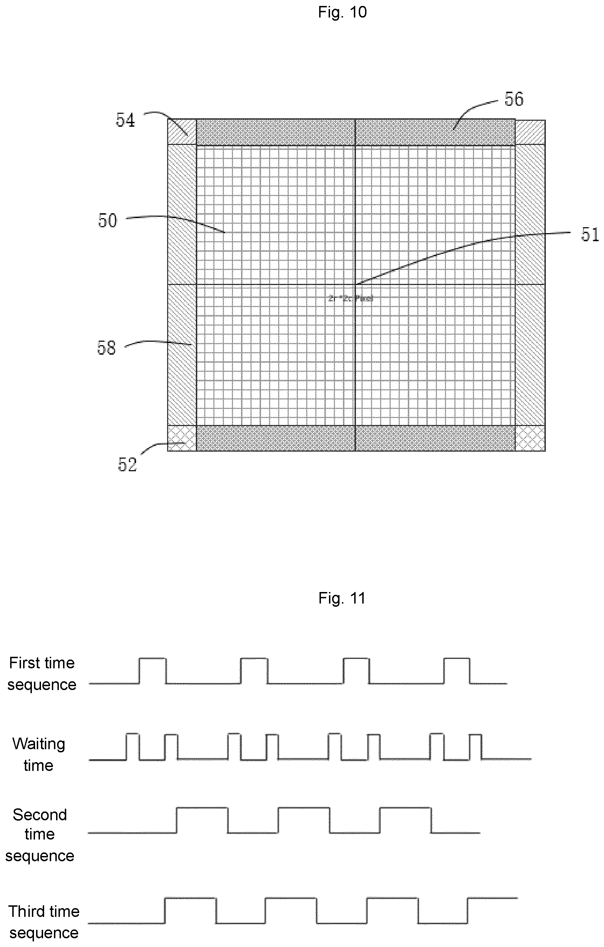

Alternatively, Fig. 11 is a timing diagram within a certain period of time captured during the testing process, the exposure time is determined by light intensity of biochemical reaction and signal-to-noise ratio, exposure time is obtained through preliminary experimental test and calculation, the continuous exposure time of each unit of core row 44 is controlled by the first time sequence, a waiting time is set before and after each exposure time (the high-level part of the first time sequence), and movement time of the manipulator 10 controlled by the second time sequence is set between two waiting times. By controlling the movement of the manipulator 10 through three time sequences, the manipulator 10 is kept in a static state during the exposure time, so that a clear and accurate image signal is obtained.

In some embodiments, data transmission of each unit of core row 44 is controlled through the third time sequence, and the data transmission time of each unit of core row 44 in the third time sequence is after the continuous exposure time of the corresponding core row 44. Therefore, data reading is carried out every time one core row 44 is immersed, joint control of immersion-reading is realized, and thus the transmission, buffering and processing loads of the system are reduced.

Alternatively, the manipulator 10 clamps the semiconductor sequencing chip 14 to move row by row along a direction vertical to the reagent tank 12, each unit of core row 44 is gradually immersed in a reagent to have biochemical reaction along with the manipulator 10, exposure is controlled to be switched to the reagent immersion region 48, an image signal is read out through the row switching and common reading unit 34, data goes up the data channel step by step through row switching circuit logic, and thus the transmission, buffering and processing loads of the system are reduced.

In some embodiments, each core 42 includes a pixel array 51, the pixel array 51 is a single pixel array 51, or the pixel array 51 is formed by stitching at least two sub-pixel arrays 50 by stitching techniques. In such a case, a semiconductor sequencing chip 14 with super-large size and super-large array may be obtained, and Fig. 10 is a semiconductor sequencing chip 14 formed by stitching a plurality of cores.

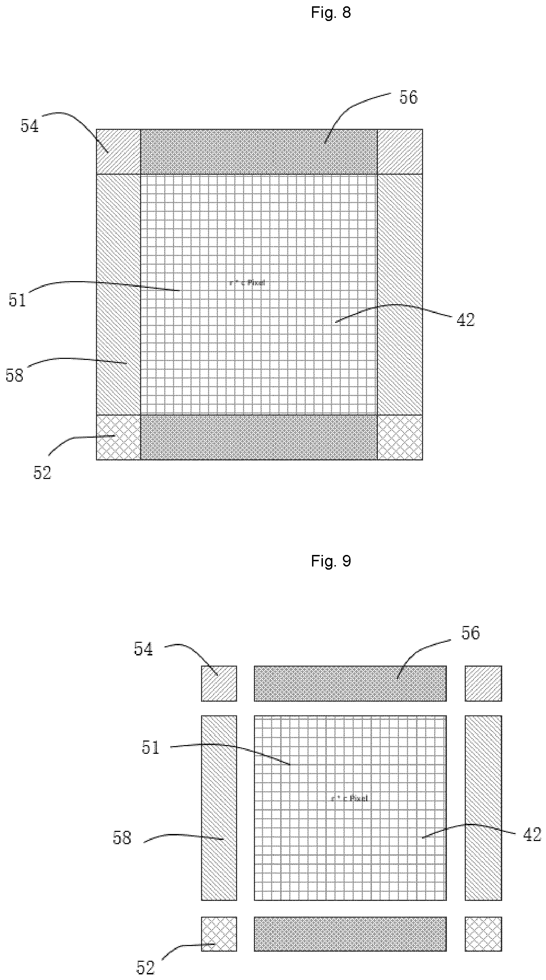

Alternatively, referring to Fig. 8, the pixel array 51 occupies the center of the core 42, while a peripheral circuit, including a phase lock loop, a decoder controller and digital buffer 52, a correlated double sampling circuit and comparator 54, a readout circuit 56 (including a digital and analog processor, a decoder and a charging mode readout circuit ), a decoder and a driver program 58, is located on the periphery of the pixel array 51 or another wafer to be bonded thereto, as shown in Fig. 9. In other implementation modes, the pixel array 51 may be formed by stitching a plurality of sub-pixel arrays 50. In an example, as shown in Fig. 10, the pixel array 51 is obtained by stitching 4 sub-pixel arrays 50 through stitching techniques, and then a chip with a larger area may be obtained. In the embodiment shown in the figure, one pixel array 51 includes r * c pixels.

To sum up, each time one immersion unit of core row 44 is immersed, data output by the core row 44 is read at least once, reading all cores in parallel is reduced to reading all core rows 44, through light-emitting reaction of the base in the two substrate reagents, type of the base may be output through simple optical signals, four bases ATCG may be represented by compressing 2 bit from 10 bit of digital quantity of each pixel, and a base sequence result may be obtained through a simplified algorithm. Therefore, the implementation of transmission, calculation and storage of the system is very simple and the cost is very low.

Some embodiments of the present disclosure further provides a system for processing data 100, configured for a system for sequencing gene, and the system for processing data 100 includes a semiconductor sequencing chip 14, a control apparatus 26 and a manipulator 10. The semiconductor sequencing chip 14 includes a processing module and a plurality of cores 42, the plurality of cores 42 are distributed in an array to form a plurality of core rows 44, the processing module is connected with the core row 44 and the control apparatus 26, the control apparatus 26 is connected with the manipulator 10, the control apparatus 26 is configured to control the manipulator 10 to immerse the semiconductor sequencing chip 14 in a reagent used for sequencing reaction in an immersion mode taking the core row 44 as an immersion unit, the reagent includes at least two substrate reagents, when the semiconductor sequencing chip 14 is immersed in the substrate reagent, corresponding bases emit light or do not emit light, and the processing module is configured to, each time when N units of core rows are immersed in the substrate reagent, N>0, read data output by the core row immersed in the reagent at least once until all the core rows are immersed in the substrate reagent.

The above system for processing data 100 immerses the semiconductor sequencing chip 14 in a reagent used for sequencing reaction in a mode taking the core row 44 as an immersion unit through joint control of immersion-reading, bases carried on the semiconductor sequencing chip 14 may show different light intensities through biochemical reaction occurring in the reagent, for example, in a substrate reagent enabling a base to emit light, only two bases in the four bases ATCG emit light or show strong light intensities, while in another substrate reagent enabling a base to emit light, the two bases do not emit light or show weak light intensity, and the light intensities of the other two bases are shown opposite. Therefore, through two substrate reagents enabling the base to emit light, the type of the base may be judged according to light intensities of the four bases ATCG therein.

The target template 11 is determined by data output by the first core row 44, and then data output by the remaining core rows 44 is simplified to obtain optical signal data of different bases. System load of sequential reading is reduced, transmission, calculation and storage of the system are simply realized, and the cost is reduced.

It is to be noted that the explanation for the implementation mode and beneficial effect of the sequencing method is also applicable to the data processing stem of the implementation mode, and no elaboration will be made here in order to avoid redundancy.

In some embodiments, after data output by the first core row 44 is read, a target template 11 is determined by the data output by the first core row 44, the target template 11 includes a signal range that whether the base emits light or not, data output by the remaining core rows 44 is simplified by the target template 11 to obtain optical signal data of different bases, and the control apparatus 26 is configured to determine the base type according to optical signal data. In such a case, the data may be simplified by the target template 11 and optical signal data is obtained, and thus the base type is determined.

Alternatively, after each immersion unit of core row 44 is immersed in two substrate reagents, all base sequence clusters/spheres loaded on the semiconductor sequencing chip 14 have biochemical reaction with the substrate reagent, after one sequencing cycle, 2 bit optical light data of the first base of all base sequence clusters/spheres is respectively read, data output by the immersion unit of core row 44 is read, one target template 11 is determined by the data output by the first core row 44, the signal range that whether the base emits light or not is defined by the target template 11, the data output by the other core rows 44 is simplified by the target template 11 to obtain and output optical signal data of different bases, in such a case, 2 bit optical signal data "00", "01", "10", or "11" may be obtained, corresponding to one of four bases "AGCT", and the control apparatus 26 reads and displays the specific base type or base sequence through the output 2 bit optical signal data.

After a plurality of sequencing cycles, optical signal data of each base of all base sequence clusters/balls loaded on the semiconductor sequencing chip 14 may be obtained in sequence, and in such a case, the base sequence of the sequencing gene may be obtained to complete the sequencing process.

In some embodiments, the reagent includes a first substrate reagent and a second substrate reagent, when the semiconductor sequencing chip 14 is immersed in the first substrate reagent or the second substrate reagent, two bases are enabled to emit light, and the other two bases do not emit light. Therefore, four different bases may be distinguished through light-emitting condition of two channels.

In some embodiments, the target template 11 includes a first signal range 13, a second signal range 15, a third signal range 17, and a fourth signal range 19, and the types of the base include a first type, a second type, a third type, and a fourth type.

The first signal range 13 indicates that the base of the first type does not emit light in both the first substrate reagent and the second substrate reagent.

The second signal range 15 indicates that the base of the second type does not emit light in the first substrate reagent and emits light in the second substrate reagent.

The third signal range 17 indicates that the base of the third type emits light in the first substrate reagent and does not emit light in the second substrate reagent.

The fourth signal range 19 indicates that the base of the fourth type emits light in both the first substrate reagent and the second substrate reagent. Therefore, the specific base type may be determined in the biochemical reaction.

In some embodiments, the processing module is further configured to:
classify the data output by the remaining core rows 44 into the signal range of the target template 11 by an intercept classification algorithm. Therefore, a sequencing error may be reduced, and a more accurate sequencing result may be obtained.

In some embodiments, simplifying processing includes binarization processing. Therefore, data computation amount may be reduced, and meanwhile, a sequencing result is accurately output.

In some embodiments, the control apparatus 26 includes an image processing module 24 connected with the semiconductor sequencing chip 14, the image processing module 24 includes a row switching and common reading unit 34, the row switching and common reading unit 34 is connected with all the core rows 44, and the control apparatus 26 is configured to control data channel switching and data reading of the core rows 44 immersed in the reagent through the row switching and common reading unit 34. In such a case, joint control of immersion-reading of the semiconductor sequencing chip 14 may be realized, and the difficulty of reading, routing, transmission, buffering and data processing of a computer system is reduced.

In some embodiments, the control apparatus 26 includes a mechanical control module 16 connected with the manipulator 10, and the mechanical control module 16 is configured to control the manipulator 10 to enable a time difference of time immersed in the reagent of each unit of core row 44 to not exceed the preset range. Therefore, the time of biochemical reaction occurring on each unit of core row 44 is uniform.

Alternatively, the mechanical control module 16 controls the manipulator 10 to clamp the semiconductor sequencing chip 14 to move, and controls the semiconductor sequencing chip 14 to be immersed in the reagent taking the core row 44 as an immersion unit. Through the mechanical control module 16, movement time of the manipulator 10 may be set, the manipulator 10 clamps the semiconductor sequencing chip 14 to be immersed in the reagent taking the core row 44 as an immersion unit, the time difference of time that all the cores 44 are immersed in the reagent is controlled to not exceed the preset range, so that the time of biochemical reaction occurring on each unit of core 44 is uniform.

In some embodiments, the control apparatus 26 includes an image processing module 24 connected with the semiconductor sequencing chip 14, the image processing module 24 includes a row switching and common reading unit 34, and the row switching and common reading unit 34 is configured to control time difference of data reading time of each unit of core row 44 not to exceed a preset range. In such a case, it may be ensured that signals obtained by all the core rows 44 are relativity uniform.

Alternatively, the image processing module 24 is controlled to set same data reading time, the time difference of time of biochemical reaction occurring in the reagent of the core row 44 immersed each time does not exceed a preset range, a signal is collected at a fixed time after each row of chip cores 42 is immersed, and it may ensure that signals obtained by all the core rows 44 are relatively uniform.

In some embodiments, before the semiconductor sequencing chip 14 is immersed in the reagent, the control apparatus 26 is further configured to divide the whole semiconductor sequencing chip 14 into a plurality of regions 48 in a manner of parallel to the reagent tank 12, each region 48 contains one unit of core row 44, and the correspondence between each region 48 and each unit of core row 44 is stored. In such a case, by dividing the plurality of regions 48, the system for sequencing gene may determine the amount and times of movement each time when the manipulator 10 clamps the semiconductor sequencing chip 14 to move.

Alternatively, the whole semiconductor sequencing chip 14 is divided into a plurality of regions 48 in a manner of parallel to the reagent tank 12, and each region 48 is the part where the manipulator 10 moves once to be immersed in the reagent. By dividing the plurality of regions 48, in the process of operating the whole semiconductor sequencing chip 14, the amount and times of movement when the manipulator 10 clamps the semiconductor sequencing chip 14 to move each time are determined.

In some embodiments, the control apparatus 26 includes an image processing module 24 connected with the semiconductor sequencing chip 14, the image processing module 24 includes a row switching and common reading unit 34, the row switching and common reading unit 34 is configured to control continuous exposure time of each unit of core row 44 by a first time sequence, and control the movement time of the manipulator 10 by a second time sequence, and a waiting time is separated before and after the continuous exposure time of each unit of core row 44 in the first time sequence from each movement time of the manipulator 10 in the second time sequence. In such a case, it is ensured that the manipulator 10 is in a static state during the exposure time, and an image signal is clearer and more accurate.

Alternatively, the exposure time is determined by light intensity of biochemical reaction and signal-to-noise ratio, exposure time is obtained through preliminary experimental test and calculation, the continuous exposure time of each unit of core row 44 is controlled by the first time sequence, a waiting time is set before and after each exposure time, and movement time of the manipulator 10 controlled by the second time sequence is set between two waiting times. The manipulator 10 is controlled to be in a static state during the exposure time through three time sequences, so that a clear and accurate image signal is obtained.

In some embodiments, the row switching and common reading unit 34 is further configured to control data transmission of each unit of core row 44 through the third time sequence, and the data transmission time of each unit of core row 44 in the third time sequence is after the continuous exposure time of the corresponding core row 44. Therefore, data reading is carried out every time one core row 44 is immersed, joint control of immersion-reading is realized, and thus the transmission, buffering and processing loads of the system are reduced.

Alternatively, the manipulator 10 clamps the semiconductor sequencing chip 14 to move row by row along a direction vertical to the reagent tank 12, each unit of core row 44 is gradually immersed in a reagent to have biochemical reaction along with the manipulator 10, exposure is controlled to be switched to the reagent immersion region 48, an image signal is read out through the row switching and common reading unit 34, data goes up the data channel step by step through row switching circuit logic, and thus the transmission, buffering and processing loads of the system are reduced.

To sum up, the sequencing method for the semiconductor sequencing chip 14 provided by the implementation mode of the present disclosure is used for a system for sequencing gene, through control of the sequencing system, the semiconductor sequencing chip 14 is immersed in the reagent for reaction with the core row 14 as an immersion unit and data is read synchronously, further, the read data is calculated, a signal range is defined for the following read data according to the target template 11 obtained according to the data of the first core row 44, thus 2 bit signals are output to determine type of the base, system load of sequential reading is reduced, transition, calculation and storage of the system are simply realized, and the cost is also reduced.

The implementation mode of the present disclosure further provides a system for sequencing gene, the explanation for the implementation mode and beneficial effect of the system for processing data of the semiconductor sequencing chip 14 is also applicable to the system for sequencing gene of the implementation mode of the present disclosure, and no elaboration will be made here in order to avoid redundancy.

In the descriptions of the specification, the descriptions made with reference to terms "some embodiments", "some embodiments", "an exemplary implementation mode", "example", "specific example", "some examples" or the like refer to that specific features, structures, materials or characteristics described in combination with the implementation mode or the example are included in at least one implementation mode or example of the present disclosure. In the specification, these terms are not always schematically expressed for the same implementation mode or example. Moreover, the specific described features, structures, materials or characteristics may be combined in a proper manner in any one or more implementation modes or examples.

According to the description of the foregoing implementation modes, the skilled in the art can clearly understand that the method in the abovementioned embodiments may be implemented by software and a necessary universal hardware platform or by hardware, although in many cases the former is a better implementation mode. Based on such an understanding, the technical solutions of the present disclosure substantially or parts making contributions to the related art may be embodied in form of software product, and the computer software product is stored in an above storage medium (such as a ROM/RAM, a magnetic disk or an optical disk), including a plurality of instructions configured to enable a terminal device (which may be a mobile phone, a computer, a server, an air conditioner, a network device or the like) to execute the method in each embodiment of the present disclosure.

The embodiments of the present disclosure have been shown or described above. However, it can be understood that the abovementioned embodiments are exemplary and should not be understood as limits to the present disclosure and those of ordinary skill in the art may make variations, modifications, replacements, transformations to the abovementioned implementation modes within the scope of the present disclosure.

## Claims

1. A sequencing method, configured for completing sequencing through a semiconductor sequencing chip, the semiconductor sequencing chip comprises a plurality of cores, the plurality of cores are arranged to form a plurality of core rows, and the method comprises:
controlling the semiconductor sequencing chip to contact a reagent used for each round of sequencing reaction in a mode taking the core row as a contact unit, and enabling a corresponding base to emit light or not to emit light when the semiconductor sequencing chip contacts the reagent;
each time when N units of core rows contact the reagent, reading data output by the core row contacting the reagent at least once until all the core rows contact the reagent, wherein N>0,,
wherein after reading data output by a first core row, determining a target template according to the data output by the first core row, and the target template comprises a signal range that whether the base emits light;
simplifying data output by a remaining core rows according to the target template to obtain optical signal data of different bases;
determining a type of the base according to the optical signal data.

2. The sequencing method as claimed in claim 1, wherein the reagent comprises a first substrate reagent and a second substrate reagent, when the semiconductor sequencing chip contacts the first substrate reagent or the second substrate, enabling two bases to emit light and the other two bases not to emit light.

3. The sequencing method as claimed in claim 2, wherein the target template comprises a first signal range, a second signal range, a third signal range, and a fourth signal range, and the types of the base comprises a first type, a second type, a third type, and a fourth type, wherein
the first signal range indicates that the base of the first type does not emit light in both the first substrate reagent and the second substrate reagent;
the second signal range indicates that the base of the second type does not emit light in the first substrate reagent and emits light in the second substrate reagent;
the third signal range indicates that the base of the third type emits light in the first substrate reagent and does not emit light in the second substrate reagent;
the fourth signal range indicates that the base of the fourth type emits light in both the first

4. The sequencing method as claimed in claim 1, wherein the sequencing method comprises:
controlling data channel switching and data reading of the core row contacting the reagent through the row switching and common reading unit, and the row switching and common reading unit is connected with all the core rows.

5. The sequencing method as claimed in claim 1, wherein the sequencing method comprises:
controlling a time difference of time that each unit of core row contacts the reagent not to exceed a preset range, or controlling a time difference of data reading time of each unit of core row not to exceed a preset range.

6. The sequencing method as claimed in claim 1, wherein before the semiconductor sequencing chip contacts the reagent, the sequencing method further comprises:
dividing a whole semiconductor sequencing chip into a plurality of regions in a manner of parallel to a reagent tank, each region contains one core row, and storing the correspondence between each region and each unit of core row.

7. The sequencing method as claimed in claim 1, wherein the semiconductor sequencing chip is controlled to contact the reagent by a manipulator, a continuous exposure time of each unit of core row is controlled by a first time sequence, a movement time of the manipulator is controlled by a second time sequence, and a waiting time is separated before and after the continuous exposure time of each unit of core row in the first time sequence from each movement time of the manipulator in the second time sequence.

8. The sequencing method as claimed in claim 7, wherein data transmission of each unit of core row is controlled by a third time sequence, and the data transmission time of each core row in the third time sequence is after the continuous exposure time of the corresponding core row.

9. The sequencing method as claimed in claim 1, wherein each core comprises a pixel array, the pixel array is a single pixel array, or the pixel array is formed by stitching at least two sub-pixel arrays.

10. A system for processing data, configured for a system for sequencing gene, wherein the system for processing data comprises a semiconductor sequencing chip, a control apparatus and a manipulator, the semiconductor sequencing chip comprises a processing module and a plurality of cores, the plurality of cores are distributed in an array to form a plurality of core rows, the processing module is connected with the core row and the control apparatus, the control apparatus is connected with the manipulator,
the control apparatus is configured to:
control the manipulator to enable the semiconductor sequencing chip to contact a reagent used for sequencing reaction in a mode taking the core row as a contact unit, and enable a corresponding base of the core row to emit light or not to emit light when the semiconductor sequencing chip contacts the reagent;
the processing module is configured to:
each time when N units of core rows contact the reagent, N>0, read data output by the core row contacting the reagent at least once until all the core rows contact the reagent.

11. The system for processing data as claimed in claim 10, wherein after data output by the first core row is read, a target template is determined according to the data output by the first core row, and the target template comprises a signal range that whether the base emits light or not;
data output by the remaining core rows is simplified according to the target template to obtain optical signal data of different bases;
the control apparatus is further configured to:
determine the type of the base on the semiconductor sequencing chip when all the core rows contact the reagent;
determine the type of the base according to the optical signal data.

12. The system for processing data as claimed in claim 10, wherein the reagent comprises a first substrate reagent and a second substrate reagent, when the semiconductor sequencing chip contacts the first substrate reagent or the second substrate reagent, two bases are enabled to emit light, and the other two bases do not emit light.

13. The system for processing data as claimed in claim 10, wherein data output by the remaining core rows are classified into the signal range of the target template by an intercept classification algorithm.

14. The system for processing data as claimed in claim 10, wherein the control apparatus comprises an image processing module connected with the semiconductor sequencing chip, the image processing module comprises a row switching and common reading unit, the row switching and common reading unit is connected with all the core rows, and the control apparatus is configured to control data channel switching and data reading of core rows in the reagent through the row switching and common reading unit.

15. The system for processing data as claimed in claim 10, wherein the control apparatus comprises a mechanical control module connected with the manipulator, and the mechanical control module is configured to control the manipulator to enable a time difference of time that each unit of core row contacts the reagent not to exceed a preset range.

16. The system for processing data as claimed in claim 10, wherein the control apparatus comprises an image processing module connected with the semiconductor sequencing chip, the image processing module comprises a row switching and common reading unit, and the row switching and common reading unit is configured to control a time difference of data reading time of each unit of core row not to exceed a preset range.

17. The system for processing data as claimed in claim 10, wherein before the semiconductor sequencing chip contacts the reagent, the control apparatus is further configured to divide the whole semiconductor sequencing chip into a plurality of regions in a manner of parallel to a reagent tank, each region contains one core row, and the correspondence between each region and each unit of core row is stored.

18. The system for processing data as claimed in claim 10, wherein the control apparatus comprises an image processing module connected with the semiconductor sequencing chip, the image processing module comprises a row switching and common reading unit, the row switching and common reading unit is configured to control continuous exposure time of each unit of core row by a first time sequence, and control movement time of the manipulator by a second time sequence, and a waiting time is separated before and after the continuous exposure time of each unit of core row in the first time sequence from each movement time of the manipulator in the second time sequence.

19. The system for processing data as claimed in claim 18, wherein the row switching and common reading unit is further configured to control data transmission of each unit of core row by a third time sequence, and data transmission time of each unit of core row in the third time sequence is after the continuous exposure time of the corresponding core row.

20. A system for sequencing gene, comprising the system for processing data as claimed in any of claims 10-19.
